# EUROPEAN PATENT APPLICATION

(11) **EP 4 613 267 A1**
(43) Date of publication of application: **10.09.2025**
(21) Application number: 23885813.8
(22) Date of filing: 01.11.2023
(51) Int. Cl.: A61K 9/48, A23L 5/00, A23P 10/35, A24D 3/06, A61J 3/07, A61K 8/11, A61K 8/31, A61K 8/37, A61K 8/73, A61K 8/92, A61K 9/56, A61K 47/06, A61K 47/14, A61K 47/36, A61K 47/44, A61Q 19/00, A61Q 90/00, B01J 13/22

(54) **WATER-FILLED CAPSULE AND MANUFACTURING METHOD THEREOF**

(30) Priority: 01.11.2022 JP 2022175360; 14.03.2023 JP 2023039340; 24.03.2023 JP 2023048468
(71) Applicant: Fuji Capsule Co., Ltd., Fujinomiya-shi, Shizuoka 418-0112 (JP)
(72) Inventor: MASUDA, Koji, Fujinomiya-shi, Shizuoka 418-0112 (JP); KONDO, Yosuke, Fujinomiya-shi, Shizuoka 418-0112 (JP); GOTO, Shungo, Fujinomiya-shi, Shizuoka 418-0112 (JP)
(74) Representative: Mathys & Squire
(86) International application number: PCT/JP2023/039428
(87) International publication number: WO 2024/096059

(57) **Abstract**

An object of the present invention is to provide a technology for a water-enclosed seamless capsule that may seal and retain the moisture of a content liquid in a capsule over a long period of time. In a water-enclosed capsule including a water-containing core containing water and an enclosing layer disposed on the outside of the water-containing core, it has become possible to seal and retain the moisture of the water-containing core in a capsule over a long period of time when the enclosing layer consists of a lipophilic composition having a specific gravity adjusted within a specific range, or when the shell thickness of the enclosing layer is at a predetermined shell thickness or more, which is a range to be retained.

## Description

### Technical Field

The present invention relates to a water-enclosed capsule including a water-containing core containing water and an enclosing layer disposed on the outside of the water-containing core, and a method for producing the water-enclosed capsule.

### Background Art

Conventionally, as a water-enclosed capsule, a water-enclosed capsule excellent in transpiration resistance of water, the capsule containing a water-containing core containing water, an inner layer disposed on the outside of the water-containing core, and an outer layer disposed on the outside of the inner layer, wherein the inner layer is composed of a lipophilic material, and wherein the outer layer is composed of a hydrophilic material in which lipophilic particles or water-insoluble particles are dispersed, is known (Patent Document 1).

In addition, with regard to a filter element for being used for tobacco products, a filter element having at least one filter body and a capsule including a core material containing a liquid medium and a water vapor-impermeable polymeric shell, wherein the liquid medium of the core material contains at least one surfactant, is known (Patent Document 2). Also, a method for producing an easily breakable raw capsule useful for incorporating into a tobacco product has been proposed (Patent Document 3).

### Prior Art Documents

### Patent Documents

Patent Document 1: Japanese Patent No. JP6250211(B)
Patent Document 2: Japanese unexamined Patent Application Publication (Translation of PCT Application) No. JP2020-527953(A)
Patent Document 3: Japanese unexamined Patent Application Publication (Translation of PCT Application) No. JP2011-512122 (A)

### Summary of the Invention

### Object to be Solved by the Invention

As a content liquid that may be formulated with a soft capsule, oil, an oil-soluble substance, or an emulsified liquid (lipophilicity + hydrophilicity) is well known. On the one hand, it is the current situation that a capsule product in which water or an aqueous solution is enclosed has not been distributed in the market as far as the applicant knows, because, even if it is possible to capsulate water or a content liquid containing water as a main component, water transpires through the shell of the capsule soon after capsulation of the water or the content liquid containing water as a main component, resulting in failure in retaining the moisture of the content liquid over a long period of time. The object of the present invention is to provide a technology for a water-enclosed seamless capsule that may seal and retain the moisture of a content liquid in a capsule over a long period of time and is water-resistant.

### Means to Solve the Object

The present inventors have conducted diligent studies so that the above object may be solved, and they have found the fact that, in the water-enclosed capsule including a water-containing core containing water and an enclosing layer disposed on the outside of the water-containing core, it becomes possible to seal and retain the moisture of the water-containing core in a capsule over a long period of time when the enclosing layer consists of a lipophilic composition having a specific gravity adjusted within a specific range or when the shell thickness of the enclosing layer is at a certain thickness to be retained or more, and the fact that it becomes possible to enhance the formulating property of the capsule when the water of the water-containing core contains a viscosity-adding agent for imparting viscosity to the water, thereby completing the present invention.

That is, the present invention is as specified by the following invention-specifying items:
[1] A water-enclosed capsule including: a water-containing core containing water; and an enclosing layer disposed on an outside of the water-containing core, wherein the enclosing layer consists of a lipophilic composition having a specific gravity of 0.79 to 1.05, and wherein a thinnest part of a shell thickness of the enclosing layer is 80 µm or more.
[2] The water-enclosed capsule according to the above [1], wherein the water-containing core contains a viscosity-adding agent for imparting viscosity to the water.
[3] The water-enclosed capsule according to the above [1], wherein the lipophilic composition contains a specific gravity-adjusting agent for adjusting the specific gravity of the lipophilic composition to 0.79 to 1.05.
[4] The water-enclosed capsule according to the above [1], wherein a melting point of the lipophilic composition is 16°C or more.
[5] The water-enclosed capsule according to the above [1], wherein the lipophilic composition contains a solid oil/fat having a melting point of 30 to 70°C.
[6] The water-enclosed capsule according to the above [1], wherein the lipophilic composition contains a liquid oil/fat having a melting point of less than 20°C.
[7] The water-enclosed capsule according to the above [1], wherein a shell layer is further provided on an outside of the enclosing layer.
[8] The water-enclosed capsule according to the above [7], wherein a moisture content of the shell layer is 20% or more.
[9] The water-enclosed capsule according to the above [7], wherein a moisture content of the shell layer is less than 20%.

In addition, the present invention is as specified by the following invention-specifying items:
[10] A method for producing the water-enclosed capsule according to any one of the above [1] to [9], the method comprising: forming a three-layered liquid drop by discharging a water-containing core from an inner nozzle, an enclosing layer from an intermediate nozzle, and a shell layer from an outer nozzle by using a triple concentric nozzle, and forming a water-enclosed pre-capsule by contacting the three-layered liquid drop with a cooling solvent.
[11] The method for producing the water-enclosed capsule according to the above [10], wherein, before drying the water-enclosed pre-capsule, the shell layer is stripped off and eliminated to leave two layers of the water-containing core containing water and the enclosing layer disposed on an outside of the water-containing core.
[12] The method for producing the water-enclosed capsule according to the above [10], wherein a three-layered water-enclosed raw capsule is prepared without drying the water-enclosed pre-capsule.
[13] The method for producing the water-enclosed capsule according to the above [10], wherein a three-layered water-enclosed dry capsule is prepared by further undergoing a step of drying the water-enclosed pre-capsule.

### Effect of the Invention

According to the present invention, a three-layered water-enclosed seamless capsule and a two-layered water-enclosed seamless capsule which exhibit little change over time in the enclosed water percentage when allowed to stand under an open atmosphere at room temperature, and which may seal and retain the moisture of the content liquid (water-containing core) in the capsule over a long period of time and are excellent in water resistance, may be provided. Also, the physical property of the enclosing layer may be set in accordance with the use of the water-enclosed capsule. In addition, the physical property of the enclosing layer disposed on the outside of the water-containing core may be set to be hard or soft depending on the oily substance constituting the enclosing layer. For example, where the enclosing layer is set to be soft, the capsule may be utilized for cosmetics, quasi-drugs, or the like as a water-enclosed raw capsule having a two-layered capsule form and having an advantage of being able to be easily ground with a finger for use.

### Mode of Carrying Out the Invention

The water-enclosed capsule of the present invention is not particularly limited as long as the capsule is a water-enclosed capsule including: a water-containing core containing water; and an enclosing layer disposed on the outside of the water-containing core, wherein the enclosing layer consists of a lipophilic composition having a specific gravity of 0.79 to 1.05, and wherein the thinnest part of the shell thickness of the enclosing layer is 80 µm or more. Suitable examples thereof may include a two-layered water-enclosed capsule and a three-layered water-enclosed capsule, but the capsule may be a multilayered water-enclosed capsule including four or more layers.

The water-containing core is not particularly limited as long as the core contains water, and the core may comprise water only, but a core containing a viscosity-adding agent for imparting viscosity to the water is preferred, and also, the core may contain various active components and various additives.

Examples of the viscosity-adding agent may include alginic acid; alginates such as sodium alginate; alginate esters such as alginic acid propylene glycol ester; celluloses such as methylcellulose, ethylcellulose, hydroxypropylmethylcellulose, hydroxypropylcellulose, and carboxymethylcellulose; gelatin; carrageenan; agar; starch; modified starch; dextran; dextrin; pullulan; glucomannan; gum arabic; furcellaran; tragacanth gum; guar gum; locust bean gum; gellan gum; xanthan gum; polyvinylpyrrolidone; polyvinyl alcohol; glucosamine; eucheuma algae; water-soluble soybean polysaccharides; welan gum; cassia gum; agrobacterium succinoglycan; an enzymatic decomposition product of guar gum; ghatti gum; arabinogalactan; curdlan; karaya gum; tamarind seed gum; psyllium seed gum; tara gum; linseed gum; konjac powder (konjac potato); hydroxypropylated guar gum; and pectin.

In the enclosing layer, examples of the oily substance constituting the lipophilic composition may include, firstly, an oily substance having a melting point of 20°C or more (hereinafter referred to as "solid oil/fat"), of which specific examples include hardened oil (oil obtained by raising the melting point by hydrogenating liquid animal/vegetable oil/fat); partially hardened oil (hardened oil obtained by partially hydrogenating liquid animal/vegetable oil/fat or a mixture of oil/fat that has not been hydrogenated and hardened oil); and waxes. Specific examples of the solid oil/fat may include hardened castor oil, hardened soybean oil, hardened rapeseed oil, hardened coconut oil, cacao butter, butter, hardened palm kernel oil, beef tallow, hardened beef tallow oil, lard, hardened lard oil, beeswax, candelilla wax, rice bran wax (rice wax), carnauba wax, Mokuro (Japan wax, urushi wax), and a mixture thereof.

Examples of the oily substance constituting the lipophilic composition may include, secondly, a liquid oily substance having a melting point of less than 20°C (hereinafter referred to as "liquid oil/fat"), which is, specifically, an animal/vegetable oil such as safflower oil, linseed oil, sesame oil, olive oil, soybean oil, mustard oil, rapeseed oil, corn oil, castor oil, evening primrose oil, palm kernel oil, jojoba oil, cottonseed oil, coconut oil, peanut oil, EPA, DHA, DPA, squalane, shark liver oil, and cod liver oil; medium-chain triglyceride (MCT); and liquid paraffin.

Examples of the oily substance constituting the lipophilic composition may also include a surfactant having higher lipophilicity (for example, a surfactant having HLB of less than 7 and a surfactant having HLB of less than 10) among surfactants such as glycerin fatty acid ester, sucrose fatty acid ester, and fatty acid acyl ester. In this case, among the surfactants, an oily substance which is solid at 20°C may be utilized as a solid oil/fat, and an oily substance which is liquid at less than 20°C may be utilized as a liquid oil/fat. With regard to the lipophilic composition that may be used for the present invention, one or more types appropriately selected from solid oils and fats and liquid oils and fats may be mixed for use, and in addition, commercially available products such as "GELUCIRE" series manufactured and sold by Gattefosse, Nissin MCT Oil, Kao "EXCEL" series, Kao "ECONA" series, Kao "EMANON" series, and Kao "RHEODOL" series may be used.

As the oily substance constituting the lipophilic composition, various oily functional components [oily components having various functions such as a nutritional function, a flavor function, and a dye function] may also be used. Examples of the oily component having a nutritional function that may be used for the present invention may include an omega fatty acid such as oleic acid, linoleic acid, linolenic acid, arachidonic acid, eicosapentaenoic acid, docosahexaenoic acid, and docosapentaenoic acid; an oily substance such as prostaglandin; an oil-soluble vitamin such as vitamin A, vitamin E, vitamin D, and vitamin K; and oils and fats containing these components. Examples of the oily component having a flavor function may include natural essential oils such as orange oil, lemon oil, perilla oil, ambrette seed oil, orris root oil, cananga oil, mustard oil, caraway oil, carrot seed oil, grapefruit oil, ginger oil, hop oil, myrtle oil, rose oil, and rosemary oil; and a synthetic or natural flavoring agent such as eugenol, ethyl caprylate, geraniol, menthol, citral, citronellal, and borneol. Examples of the oily component having a dye function may include an oily colorant such as annatto dye, carotene, and oleoresin. Naturally, these may be used alone or in combination.

The lipophilic composition may contain an oil-insoluble component. As the oil-insoluble component, any component that may be mixed with an oily component may be used. As the oil-insoluble component that may be mixed with an oily component, for example, an inorganic powder such as titanium dioxide, calcium carbonate, magnesium carbonate, magnesium oxide, calcium stearate, magnesium stearate, (fine particles) silicon dioxide, and aluminum oxide may be used.

A specific gravity-adjusting agent for adjusting the specific gravity of the lipophilic composition to a predetermined range may be used. The specific gravity-adjusting agent is a substance that may be dissolved or mixed with the lipophilic composition and has a specific gravity different from the specific gravity of the lipophilic composition before the specific gravity-adjusting agent is added thereto. As the specific gravity-adjusting agent, an oily substance having a specific gravity of 1 or more such as SAIB (Sucrose Acetate Isobutyrate), wood rosin glycerin ester, and sucrose octaacetate may be suitably used. In addition, an inorganic powder such as titanium dioxide, calcium carbonate, magnesium carbonate, magnesium oxide, calcium stearate, magnesium stearate, (fine particles) silicon dioxide, and aluminum oxide may also be used as the specific gravity-adjusting agent as long as the specific gravity may be adjusted by mixing the inorganic powder with the lipophilic composition before the specific gravity-adjusting agent is added thereto. Furthermore, animal/vegetable oil, synthetic oil, ester oil, mineral oil, and the like that are acceptable for the purpose of use of the present invention may also be used as the specific gravity-adjusting agent as long as the specific gravity may be adjusted by adding them to the lipophilic composition before the specific gravity-adjusting agent is added thereto.

Suitable examples of the specific gravity of the enclosing layer in the present invention may include 0.79 to 1.05, 0.79 to 1.03, 0.79 to 1.0, 0.795 to 1.05, 0.795 to 1.03, 0.795 to 1.0, 0.8 to 1.05, 0.8 to 1.03, 0.8 to 1.0, 0.83 to 1.05, 0.83 to 1.03, 0.83 to 1.0, 0.85 to 1.05, 0.85 to 1.03, and 0.85 to 1.0, and of these, the most suitable example may be 0.80 to 1.0. It has been confirmed that the water-enclosed capsule may be formulated by using a lipophilic composition having low specific gravity [for example, a mixture of the squalane and the hardened oil; the specific gravity of the enclosing layer: 0.795] and a lipophilic composition having high specific gravity [for example, a mixture of the SAIB and the hardened oil; the specific gravity of the enclosing layer: 1.03] as enclosing layers.

With regard to the shell thickness of the enclosing layer, what is important is the thickness of the thinnest part (shell thickness of the thinnest part), and as long as the shell thickness of the thinnest part is kept at a minimum level or more, which is, for example, 80 µm or more, preferably 90 µm or more, 100 µm or more, 110 µm or more, 120 µm or more, 150 µm or more, 180 µm or more, 210 µm or more, 240 µm or more, 270 µm or more, 300 µm or more, 350 µm or more, 400 µm or more, 450 µm or more, or 500 µm or more, the shell thickness may be uneven.

A three-layered water-enclosed capsule may be formed by further providing a shell layer on the outside of the enclosing layer. The shell layer may be any layer that is normally used as a shell of a seamless capsule, and examples of the shell layer may include a shell layer containing, as a base, gelatin, casein, zein, pectin, and a derivative thereof; alginic acid or a salt thereof; agar; gellan gum; tragacanth gum; guar gum; locust bean gum; carrageenan; tamarind; mannan; hemilose; starch; or chitosan. In addition, the shell layer may contain, in addition to the base, a plasticizer such as glycerin, sorbitol, propylene glycol, and polyethylene glycol; a pH-adjusting agent such as sodium phosphate; a chelating agent such as trisodium citrate and sodium metaphosphate; a gelation-promoting agent such as calcium lactate and potassium chloride; a surfactant such as polyglyceryl fatty acid ester and lecithin; a sweetener; a flavor; a preservative; a coloring agent; or the like. Furthermore, the shell layer may contain an additive such as a natural dye, a synthetic dye, various sweeteners, a preservative, a moisture activity-reducing agent, and a pH-adjusting agent. The three-layered water-enclosed capsule may be classified into a dry capsule and a raw capsule depending on the moisture content of the shell layer.

As a shell liquid used for the shell layer, a shell liquid having a viscosity of 300 mPa•s or less at 85°C as measured by using a C-type viscometer (manufactured by Toki Sangyo Co., Ltd., VISCOMETER TVC-7) with rotor No. 1 (upper limit: 500 mPa•s) is preferred.

The method for producing the water-enclosed capsule of the present invention is not particularly limited as long as it is a method comprising: discharging the water-containing core from an inner nozzle, the enclosing layer from an intermediate nozzle, and the shell layer from an outer nozzle by using a triple concentric nozzle to form a three-layered liquid drop, and contacting the three-layered liquid drop with a cooling solvent set at, for example, 0 to 25°C to form a water-enclosed pre-capsule, and examples of the cooling solvent may include a cooling oil such as an MCT being a fatty acid such as capric acid and caprylic acid; liquid paraffin; a vegetable oil such as sunflower oil and safflower oil; and a mixture thereof, and further include a low-temperature gas such as air, helium, nitrogen, and argon.

Where the shell layer is stripped off and eliminated before the water-enclosed pre-capsule is dried, a two-layered water-enclosed capsule including two layers of a water-containing core containing water and an enclosing layer disposed on the outside of the water-containing core may be produced. When the shell layer is stripped off and eliminated before the water-enclosed pre-capsule is dried, since the shell layer is in a soft state, a simple tool corresponding to a finger, a medicine spoon, and the like may be used to easily peel off the shell layer.

Where the water-enclosed capsule of the present invention is a two-layered water-enclosed capsule, the capsule may be roughly sorted into a solid capsule and a raw capsule depending on the hardness of the enclosing layer, and a capsule including a hard enclosing layer and a capsule including a soft enclosing layer may be, after a fashion, classified as a solid capsule and a raw capsule, respectively, but since the boundary line on the difference in physical properties of them may not be clearly defined, it has been decided to newly use a rheometer to measure the hardness for each enclosing-layer prescription and compare the maximum loads thereof. In the present invention, a two-layered water-enclosed capsule formed with an enclosing layer having a maximum load of 5 kg or less and a two-layered water-enclosed capsule formed with an enclosing layer having a maximum load of more than 5 kg as measured by the following [Measurement Method] by using the following [Apparatus Used for Measurement], when the adapter was pushed in to 3 mm with the rheometer, are classified as a two-layered water-enclosed raw capsule (two-layered raw capsule) and a two-layered water-enclosed solid capsule (two-layered solid capsule), respectively, for convenience.

### [Measurement Method]

150 g of the enclosing layer is prepared in a 200 mL glass beaker, and the layer is dissolved and stirred well in a water bath at 60°C (75°C where hardened oil e is contained). 50 g of the solution is put in a plastic bottle and refrigerated for 3 hours or more to cool and solidify the solution, which was then allowed to stand until the temperature returned to room temperature, and measured for the maximum load at the point when the center of the plastic bottle was pushed in to 3 mm with the adapter on the rheometer.

### [Apparatus Used for Measurement]

SUN RHEO METER CR-3000 EX-L (Sun Scientific Co., Ltd.)
Mode: MODEl (Depth); Adapter: No. 1 φ 10 mm
REAL/HOLD: HOLD; Penetration distance: 3.0 mm
Compression/Tension: PRESS; Table movement speed: 20.0 mm/min
Maximum load cell stress: 200 N

In order to produce a two-layered solid capsule, a solid oil/fat, or a solid oil/fat and a liquid oil/fat are used as an example of the lipophilic composition of the enclosing layer, and the melting point of the enclosing layer (actual measurement value) is, for example, 37°C or more and the specific gravity of the enclosing layer is, for example, 0.82 to 1.05. On the other hand, in order to produce a two-layered raw capsule, a mixed oil/fat of a solid oil/fat and a liquid oil/fat is used as an example of the lipophilic composition of the enclosing layer, and the melting point of the enclosing layer (actual measurement value) is, for example, 16 to 36.8°C and the specific gravity of the enclosing layer is, for example, 0.79 to less than 0.82. In addition, the two-layered raw capsule is normally an easily breakable capsule. It has been found that, the melting point of the enclosing layer, which is a mixed oil/fat of a solid oil/fat and a liquid oil/fat, exhibits an almost single peak, not a double peak of the melting point of the solid oil/fat and the melting point of the liquid oil/fat.

In the method for measuring the melting point, a differential scanning calorimeter "DSC7020" (manufactured by Hitachi High-Tech Science Corporation) was used under a measurement-starting temperature of 0°C and a temperature-increase rate of 5°C/min, and the endothermic peak was defined as the actual measurement value of the melting point.

The melting point of the oily substance constituting the lipophilic composition is not particularly limited, but where the water-enclosed capsule is a two-layered capsule, the capsule may be roughly sorted into a solid capsule and a raw capsule depending on the texture of the lipophilic composition. That is, in the case of a two-layered solid capsule, the lipophilic composition may have a melting point of 20°C or more, and preferably 30°C or more, and preferably have a melting point of 100°C or less, preferably 80°C or less, and more preferably 70°C or less, and of these, the most suitable example may be a lipophilic composition having a melting point of 30°C to 70°C. The lipophilic composition may be appropriately adjusted by mixing a plurality of oily substances such as hardened oils having different melting points together, or adding liquid animal/vegetable oil. On the other hand, where the texture of the water-enclosed capsule is a raw capsule, by way of example, a mixed oil/fat of the solid oil/fat and a liquid oil/fat having a melting point of less than 20°C is used. For example, in the case of a water-enclosed capsule in which the melting point of the mixed oil/fat of the enclosing layer is 16°C, even if being liquid at room temperature, the capsule is kept in a half-raw state under refrigerated conditions and, accordingly, assuming distribution and use under refrigerated conditions, the capsule may also be regarded as a two-layered raw capsule.

On the one hand, where the water-enclosed capsule is a three-layered capsule, the capsule may be roughly sorted into a raw capsule and a dry capsule mainly depending on the texture of the shell. In the case of a three-layered water-enclosed raw capsule (three-layered raw capsule), the distinct characteristic is that a three-layered raw capsule is prepared from the water-enclosed pre-capsule formed by discharging the water-containing core from an inner nozzle, the enclosing layer from an intermediate nozzle, and the shell layer from an outer nozzle by using the triple concentric nozzle to form a three-layered liquid drop and contacting the three-layered liquid drop with a cooling solvent, without undergoing a step of drying the shell. The (step) "without undergoing a step of drying the shell" refers to producing a three-layered water-enclosed capsule without using a known means for drying capsules such as spray drying, freeze drying, air drying, vacuum drying, stationary drying, and vacuum vibration drying, but stopping the step of drying the shell (drying-stop step) before the moisture content ratio (moisture content) of the shell of the water-enclosed pre-capsule reaches, for example, less than 20%, less than 25%, less than 30%, less than 45%, less than 70%, or less than 90% may also be included for convenience. Examples of the moisture content of the shell of the three-layered raw capsule may include 20% or more, preferably 25% or more, more preferably 30% or more, more preferably 45% or more, further preferably 70% or more, and further more preferably 90% or more.

Each shell of the three-layered raw capsule that has not undergone the step of drying the shell is soft, and the moisture content of the shell may be measured by using an infrared moisture meter FD-720 (manufactured by Kett Electric Laboratory Co., Ltd.) under a condition of 1 g of the shell/105°C/15 minutes, for example.

In other words, the three-layered raw capsule may be produced and stored by the method including the following steps (a) to (c):
(a) forming a three-layered liquid drop by discharging a water-containing core from an inner nozzle, an enclosing layer from an intermediate nozzle, and a shell layer from an outer nozzle by using a triple concentric nozzle;
(b) forming a water-enclosed pre-capsule by contacting the three-layered liquid drop with a cooling solvent; and
(c) immersing the water-enclosed pre-capsule in a capsule-storing liquid.

The three-layered water-enclosed capsule may be washed with a detergent or running water as necessary in order to remove the cooling solvent adhering around the capsule.

Examples of the capsule-storing liquid may include an aqueous solvent containing at least one kind of water selected from tap water, well water, distilled water, purified water, ion-exchanged water, ultrapure water, deep ocean water, mineral water, and the like. As the moisture content of the shell is high, for example, where use for cosmetics is assumed, a preservative such as phenoxyethanol or an antibacterial agent such as 1,3-butylene glycol may be added to the shell, the storing liquid, or the like in order to prevent decomposition of the shell. The three-layered raw capsule thus obtained is excellent in that the capsule may be immersed in the storing liquid and transported, and the capsule may seal and retain the contained moisture or the contained water-soluble component in a capsule over a long period of time in a state of a raw capsule even during storage and transportation. Therefore, examples of the use of the three-layered raw capsule include use of the capsule in a form in which the capsule is mixed and dispersed in a product rich in moisture, and the fields in which the capsule is used are assumed to range widely over foods, medicines, quasi-drugs, cosmetic compositions such as cosmetics, skin-care products, beverages, pet foods, luxury items, and the like.

On the one hand, a three-layered dry water-enclosed capsule (three-layered dry capsule) may be prepared by drying the water-enclosed pre-capsule by using a known means for drying capsules such as spray drying, freeze drying, air drying, vacuum drying, stationary drying, and vacuum vibration drying. Examples of the moisture content of the shell of the three-layered dry capsule may include less than 20%, preferably 15% or less, more preferably 10% or less, and further preferably 8% or less.

In other words, the three-layered dry capsule may be produced by the method including the following steps (a) to (c) :
(a) forming a three-layered liquid drop by discharging a water-containing core from an inner nozzle, the enclosing layer from an intermediate nozzle, and the shell layer from an outer nozzle by using a triple concentric nozzle;
(b) forming a water-enclosed pre-capsule by contacting the three-layered liquid drop with a cooling solvent; and
(c) producing a three-layered dry capsule by drying the water-enclosed pre-capsule.

In the case of the three-layered dry capsule, where, for example, an easily crackable capsule used for a tobacco is assumed, the hardness of the capsule shell may be 0.5 to 40 N, for example, and preferably 0.5 to 20 N, and the thickness of the capsule shell may be 20 to 500 µm, for example, and preferably 40 to 200 µm, from the viewpoint of the productivity, the easy-cracking property, and the like, and the diameter of the capsule may be 0.5 to 15 mm, for example, and preferably 1 to 8 mm, and the shell percentage of the capsule may be 5 to 30%, for example, from the viewpoint of easy holding, the internal volume, and the like.

The three-layered dry capsule may be easily cracked with a finger, and the snapping sound and a feel generated when the capsule is cracked are pleasant. Therefore, if a capsule containing a flavor or the like is embedded inside a filter that comes with a tobacco or inside a filter to be attached to a tobacco, the sound and the feel of the capsule being crushed and the fragrance may be obtained by crushing the capsule with a finger during smoking. In addition, if a capsule containing a flavor is placed on a greeting card, the recipient may crush the capsule to obtain the sound, the feel, and the fragrance. The capsule may also be applied to medicines, health foods, foods, cosmetics, daily necessities such as cleaning supplies, and the like. The three-layered dry shell capsule may contain, in addition to the easily crackable capsule used for a tobacco as described above, a capsule suitable for oral ingestion.

Examples of the method for determining the enclosed water stability of the capsule of the present invention may include, in the case of a two-layered solid capsule, a method in which the mass of the capsule that has been allowed to stand under an open atmosphere at 22°C at 18 to 45% RH (or at 5°C at 70% RH, or at 25°C at 8% RH) is measured to follow the change over time in the enclosed water percentage, and the capsule is determined to have enclosed water stability if it is 75% or more, preferably 85% or more, and more preferably 90% or more after 4 weeks, and the capsule is determined to have enclosed water stability if it is 50% or more, preferably 75% or more, more preferably 78% or more, further preferably 80% or more, and particularly preferably 90% or more after 8 weeks.

In the case of the three-layered dry capsule which is an easily crackable capsule used for a tobacco, if the sound and the feel generated when the capsule is crushed with a finger are satisfactory even one month (1M) after the capsule is dried and therefore the sound and the feel of the capsule being cracked may be enjoyed, it may be determined that the moisture or the water-soluble component in the water-containing core has not transferred to the shell part and therefore the capsule has enclosed water stability.

Examples of the method for evaluating the water resistance of the capsule of the present invention may include a method in which the transfer of the dye in the water-containing core is visually confirmed when the water-enclosed capsule is immersed for storage in the storing liquid. If the moisture or the water-soluble component of the water-containing core is sealed and stably retained in the raw capsule, the storing liquid is in the colorless state and there is no change in the color tone, but when the moisture or the water-soluble component in the water-containing core has transferred to the storing liquid part through the shell, the storing liquid is colored blue, and it may be visually confirmed that the moisture or the water-soluble component of the water-containing core is not stably retained.

Hereinafter, the present invention will be specifically described with reference to Examples, but the present invention is in no way limited thereto.

### Examples

### [Production of Water-enclosed Capsule]

A three-layered water-enclosed pre-capsule including a water-containing core, an enclosing layer covering the water-containing core, and a shell layer covering the enclosing layer was produced by discharging a shell liquid consisting of the composition shown in Table 1 from an outermost nozzle, a water-containing core liquid consisting of the composition shown in Tables 2 and 3 from an innermost nozzle, and an enclosing layer liquid consisting of the composition shown in Tables 2 and 3 from an intermediate nozzle by using a triple concentric nozzle (manufactured by Fuji Capsule Co., Ltd.) to form a three-layered liquid drop, and contacting the three-layered liquid drop with a cooling solvent consisting of MCT to cure the shell liquid.

A two-layered water-enclosed capsule was produced by stripping off the gelatin shell layer, which was the outermost layer of the three-layered water-enclosed pre-capsule, without undergoing the step of drying. It has been confirmed that water may be enclosed as long as the enclosing layer of the two-layered water-enclosed capsule is present as the outer layer and, therefore, the gelatin shell layer is not necessarily required.

**[Table 1]**

| Ingredient name | Amount (parts by mass) |
|---|---|
| Gelatin (jelly strength: 200) | 100 |
| Glycerin | 15 |
| Purified water | Proper amount |

**[Table 2]**

| | | | Example 1 | Example 2 | Example 3 | Example 4 | Example 4-1 | Example 5 | Example 6 | Example 7 | Comparative Example 1 | Comparative Example 2 |
|---|---|---|---|---|---|---|---|---|---|---|---|---|
| | Capsule structure | | Two-layered capsule | Two-layered capsule | Two-layered capsule | Two-layered capsule | Three-layered capsule | Two-layered capsule | Two-layered capsule | Two-layered capsule | - | - |
| Water-containing core | Amount filled | | 100 mg | 200 mg | 200 mg | 100 mg | 100 mg | 100 mg | 100 mg | 100 mg | 100% of water is enclosed in polyethylene bag | 100% of water is enclosed in rubber balloon (water balloon) |
| | Water content | | 98.5% | 98.5% | 98.5% | 98.5% | 98.5% | 98.5% | 98.5% | 100.0% | | |
| | Viscosity-adding agent | | Sodium alginate | Sodium alginate | Sodium alginate | Sodium alginate | Sodium alginate | Sodium alginate | Sodium alginate | - | | |
| Enclosing layer | Actual measurement value of melting point of solid oil/fat | | a | a | a | - | - | - | - | a | | |
| | | | - | - | - | b | b | - | - | - | | |
| | | | - | - | - | - | - | c | - | - | | |
| | a:44.2°C | | - | - | - | - | - | - | d | - | | |
| | b:35.3°C | | | | | | | | | | | |
| | c:38.6°C | | | | | | | | | | | |
| | d:39.7°C | | | | | | | | | | | |
| | Specific gravity-adjusting agent | | - | - | SAIB | SAIB | SAIB | - | - | - | | |
| | Specific gravity of encapsulating layer | | 0.890 | 0.890 | 0.945 | 0.945 | 0.945 | 0.895 | 0.895 | 0.890 | | |
| | Shell thickness of thinnest part | | 500 µm or more | 500 µm or more | 500 µm or more | 500 µm or more | 500 µm or more | 500 µm or more | 500 µm or more | 500 µm or more | | |
| Shell layer | Shell | | None | None | None | None | Gelatin-based | None | None | None | | |
| Change over time in enclosed water percentage | Allowed to stand under open atmosphere at 22°C at 18 to 45% RH | 4W | 99.7% | 99.3% | 99.1% | 97.2% | 96.9% | 99.4% | 98.6% | 99.3% | 97.3% | 74.6% |
| | | 8W | 98.8% | 98.7% | 98.1% | 94.7% | 94.2% | 98.2% | 96.9%. | 98.3% | 93.9% | 45.1% |
| | | 12W | 98.1% | 98.2% | 97,4% | 92.4% | 91.6% | 97.4% | 95.5% | 97.5% | 91.1% | Balloon went off |
| | | 16W | 97.3% | 97.4% | 96.7% | 90.3% | 89.3% | 96.4% | 94.1% | 96.8% | 87.4% | - |
| | Allowed to stand under open atmosphere at 5°C at 70% RH | 4W | 100.0% | 100.0% | - | - | - | - | - | - | - | - |
| | | 8 W | 100.0% | 99.9% | - | - | - | - | - | - | - | - |
| | | 12 W | 99.9% | 99.8% | - | - | - | - | - | - | - | - |
| | | 16 W | 99.8% | 99.7% | - | - | - | - | - | - | - | - |
| | Allowed to stand under open atmosphere at 25°C at 8% RH | 4 W | 98.3% | - | - | - | - | - | - | - | - | - |
| | | 8 W | 96.7% | - | - | - | - | - | - | - | - | - |
| | | 12 W | 95.5% | - | - | - | - | - | - | - | - | - |
| | | 16 W | 94.0% | - | - | - | - | - | - | - | - | - |

| | | | | | | | | | | | | |
|---|---|---|---|---|---|---|---|---|---|---|---|---|
| Polyethylene bag: made of low-density polyethylene resin; thickness: 0.06 mm Rubber balloon (water balloon): made of natural rubber; thickness: 0.10 mm SAIB: Sucrose Acetate Isobutyrate (Eastman Chemical) Apparatus used for measurement of shell thickness of thinnest part: High-resolution 3D X-ray microscope "NANO3DX" (manufactured by Rigaku Corporation) Apparatus used for measurement of melting point of oil/fat: Differential scanning calorimeter "DSC7020" (manufactured by Hitachi High-Tech Science Corporation); measurement start: 0°C, temperature increase rate: 5°C/min | | | | | | | | | | | | |

**[Table 3]**

| | | | Example 8 | Example 9 | Example 10 | Example 11 | Example 12 | Comparative Example 3 |
|---|---|---|---|---|---|---|---|---|
| | Capsule structure | | Two-layered capsule | Two-layered capsule | Two-layered capsule | Two-layered capsule | Two-layered capsule | Two-layered capsule |
| Water-containing core | Amount filled | | 100 mg | 100 mg | 100 mg | 100 mg | 100 mg | 100 mg |
| | Water content | | 98.5% | 98.5% | 98.5% | 98.5% | 98.5% | 98.5% |
| | Viscosity-adding agent | | Sodium alginate | Sodium alginate | Sodium alginate | Sodium alginate | Sodium alginate | Sodium alginate |
| Enclosing layer | Actual measurement value of melting point of solid oil/fat a:44.2°C | | a | a | a | a | a | a |
| | Specific gravity-adjusting agent | | - | - | - | - | - | SAIB |
| | Specific gravity of enclosing layer | | 0.890 | 0.890 | 0.890 | 0.890 | 0.890 | 0.945 |
| | Film thickness of thinnest part | | 700 µm | 450 µm | 250 µm | 120 µm | 90 µm | 70 µm |
| Shell layer | Shell | | None | None | None | None | None | None |
| Change over time in enclosed water percentage | Allowed to stand under open atmosphere at 22°C at 18 to 45% RH | 4W | 99.2% | 98.8% | 98.5% | 97.7% | 97.2% | 49.2% |
| | | 8W | 98.0% | 97.8% | 96.9% | 95.5% | 94.8% | 24.8% |
| | | 12 W | 96.9% | 96.7% | 95.1% | 93.2% | 92.0% | 14.0% |
| | | 16W | 95.9% | 95.4% | 93.3% | 90.9% | 89.2% | 6.4% |

### [Examples 1 to 7]

In the water-containing cores of Examples 1 to 6, the water is thickened with KIMICA ALGIN IL-2 (manufactured by KIMICA Corporation), which is sodium alginate, to improve the filling property. Water may be enclosed in the enclosing layers if the enclosing layers are based on four solid oils and fats and the thicknesses of the enclosing layers are more than 70 µm. In Examples 3, 4, and 4-1, SAIB (manufactured by Eastman Chemical Company), which is a specific gravity-adjusting agent, is added to the solid oil/fat to improve the filling property.

In the enclosed water percentage test shown in Table 2, the changes over time in the enclosed water percentage were measured after the capsule was allowed to stand for 4 weeks, 8 weeks, 12 weeks, and 16 weeks under an open atmosphere at 22°C at 18 to 45% RH (or at 5°C at 70% RH, or at 25°C at 8% RH). As a reference and an objective evaluation criterion for the enclosed water percentage, 100% of water was enclosed in Comparative Example 1 [polyethylene bag: made of low-density polyethylene resin, thickness: 0.06 mm] and Comparative Example 2 [rubber balloon (water balloon): made of natural rubber, thickness: 0.10 mm], respectively and the change over time in the enclosed water percentage was measured. Comparative Example 1 was superior to Comparative Example 2 for the enclosed water percentage, and Examples 1 to 7 had enclosed water percentages that were almost the same as or superior to that of Comparative Example 1.

Example 4 was obtained by the process of obtaining the capsule of Example 4-1 by seamless filling with three layers, and then stripping off the gelatin shell layer, which was the outer layer, to form a two-layered water-enclosed capsule without undergoing the step of drying the shell. Water may be enclosed as long as the enclosing layer is present as the outer layer and therefore, the shell layer (gelatin) is not necessarily required, and presence or absence of the shell layer is appropriately selected depending on use of the product. In addition, from the result of the change over time in the enclosed water percentage in Examples 4 and 4-1, there is no difference in the enclosed water percentage between the two-layered and three-layered structures.

### [Examples 8 to 12]

In Examples 8 to 12 and Comparative Example 3, in order to grasp the thinnest shell thickness of the enclosing layer that might enclose water, two-layered capsules having different thinnest shell thicknesses of the enclosing layers from 700 to 70 µm were produced, and the changes over time in the enclosed water percentage after the capsule was allowed to stand for 4 weeks, 8 weeks, 12 weeks, and 16 weeks under an open atmosphere at 22°C at 18 to 45% RH were measured. For the measurement of the shell thickness of the thinnest part of the enclosing layer, a high-resolution 3D X-ray microscope "NANO3DX" (manufactured by Rigaku Corporation) was used, and a non-destructive cross-sectional image was taken to confirm the shell thickness.

On the one hand, where the shell thickness of the thinnest part of the enclosing layer was 70 µm (Comparative Example 3), the enclosed water percentage significantly decreased and therefore the capsule was not suitable for the water-enclosed capsule, but, if the shell thickness of the thinnest part was more than 70 µm, a satisfactory enclosed water percentage was retained.

Each water-enclosed capsule was produced in accordance with the prescription shown in following Table 4.

**[Table 4]**

| | | | Comparative Example 4 | Comparative Example 5 | Example 13 | Example 14 | Example 15 | Example 16 | Example 17 | Example 18 | Example 19 | Comparative Example 6 | Comparative Example 7 |
|---|---|---|---|---|---|---|---|---|---|---|---|---|---|
| | Capsule structure | | Three-layered capsule | Three-layered capsule | Two-layered capsule | Two-layered capsule | Two-layered capsule | Two-layered capsule | Two-layered capsule | Three-layered capsule | Three-layered capsule | Two-layered capsule | Three-layered capsule |
| Water-containing core | Amount filled | | 100 mg | 100 mg | 100 mg | 100 mg | 100 mg | 100 mg | 100 mg | 100 mg | 100 mg | 100 mg | 100 mg |
| | Water content | | 98.5% | 98.5% | 98.5% | 98.5% | 98.5% | 98.5% | 98.5% | 98.5% | 98.5% | 98.5% | 98.5% |
| | Viscosity-adding agent | | Sodium alginate | Sodium alginate | Sodium alginate | Sodium alginate | Sodium alginate | Sodium alginate | Sodium alginate | Sodium alginate | Sodium alginate | Sodium alginate | Sodium alginate |
| Enclosing layer | Actual measurement value of melting point of solid oil/fat | | - | - | a | a | a | a | a | a | a | a | a |
| | | | - | e | - | - | e | - | - | - | - | - | - |
| | a:44.2°C | | | | | | | | | | | | |
| | e:64.8°C | | | | | | | | | | | | |
| | Liquid oil/fat | | Squalane | Squalane | Squalane | Squalane | Squalane | Squalane | - | - | - | - | - |
| | Specific gravity-adjusting agent | | - | - | - | - | - | - | SAIB | SAIB | SAIB | SAIB | SAIB |
| | Specific gravity of enclosing layer | | 0.775 | 0.785 | 0.795 | 0.815 | 0.825 | 0.835 | 0.990 | 0.990 | 1.030 | 1.065 | 1.065 |
| | Shell thickness of thinnest part | | - | - | 500 µm or more | 500 µm or more | 500 µm or more | 500 µm or more | 500 µm or more | 500 µm or more | 500 µm or more | - | - |
| | State of enclosing layer | | - | - | Raw | Raw | Solid | Solid | - | - | - | - | - |
| | Maximum load measured with rheometer | | - | - | 0.75 kg | 3.42 kg | 5.81 kg | 7.18 kg | - | - | - | - | - |
| Shell layer | Shell | | Gelatin-based | Gelatin-based | None | None | None | None | None | Gelatin-based | Gelatin-based | None | Gelatin-based |
| Change over time in enclosed water percentage | Allowed to stand under open atmosphere at 22°C at 18 to 45% RH | 4W | Difficult to formulate | Difficult to formulate | 90.9% | 96.1% | 99.5% | 97.7% | 90.5% | 90.2% | 77.2% | 37.2% | 36.5% |
| | | 8W | | | 82.2% | 92.3% | 98.7% | 95.7% | 80.1% | 79.8% | 52.7% | 2.2% | 2.4% |

### [Apparatus Used for Measurement with Rheometer]

SUN RHEO METER CR-3000 EX-L (Sun Scientific Co., Ltd.)
MODE: MODEl (Depth); Adapter: No. 1 φ 10 mm
REAL/HOLD: HOLD; Penetration distance: 3.0 mm
Compression/Tension: PRESS; Table movement speed: 20.0 mm/min
Maximum load cell stress: 200 N

### (Examples 13 and 14)

In the water-enclosed capsules of Examples 13 and 14, an oily substance in which solid oil/fat and squalane, which was a liquid oil/fat considered to have a melting point of -38°C, were mixed together was used for the enclosing layer, and the specific gravities of the enclosing layers were set to low specific gravities of 0.795 and 0.815, respectively. The maximum loads measured with the rheometer were 0.75 kg in Example 13 and 3.42 kg in Example 14, and two-layered water-enclosed raw capsules in which the enclosing layers were set to be soft were successfully obtained. The data after 8 weeks in the test of change over time in the enclosed water percentage show that capsules of both Examples 13 and 14 have retained enclosed water percentages higher than that of Comparative Example 2 (rubber balloon (water balloon)). Therefore, it was confirmed that a two-layered raw capsule in which the specific gravity of the enclosing layer was 0.795 to 0.815 retained an excellent enclosed water percentage.

### (Examples 15 and 16)

In the water-enclosed capsules of Examples 15 and 16, an oily substance in which solid oil/fat and squalane, which was a liquid oil/fat, were mixed together was used for the enclosing layer, and the specific gravity of the enclosing layer was set to a little high specific gravity of 0.825 or 0.835. The maximum loads measured with the rheometer were 5.81 kg in Example 15 and 7.18 kg in Example 16, and two-layered water-enclosed solid capsules in which the enclosing layers were set to be relatively hard were obtained.

### (Example 17)

A two-layered solid capsule, in which the specific gravity of the lipophilic composition in the enclosing layer was set to a high specific gravity of 0.99 by using SAIB as a specific gravity-adjusting agent, was prepared for Example 17. The data after 8 weeks in the test of change over time in the enclosed water percentage show that the capsule have retained a water-enclosed percentage higher than that of Comparative Example 2 (rubber balloon (water balloon)).

### [Preparation of Three-layered Water-enclosed Dry Capsule]

### (Examples 18 and 19)

With regard to Example 18, a capsule in which the specific gravity of the enclosing layer was set to a high specific gravity of 0.99 was prepared as a three-layered dry capsule having a gelatin-based shell. With regard to Example 19, a capsule in which the specific gravity of the enclosing layer was set to 1.03, which was higher than the specific gravity of the enclosing layer of Example 18, was prepared as a three-layered dry capsule having a gelatin-based shell.

For both Examples 18 and 19, the data after 4 weeks in the test of change over time show that it is 75% or more, and the data after 8 weeks in the test of change over time in the enclosed water percentage show that it is 50% or more, which means that the capsules of both Examples 18 and 19 each have retained an enclosed water percentage higher than that of Comparative Example 2 (rubber balloon (water balloon)).

### (Comparative Examples 4 and 5)

A sample in which the specific gravity of the lipophilic composition in the enclosing layer was set to a low specific gravity of 0.775 by using squalane as a specific gravity-adjusting agent was used for Comparative Example 4, and a sample in which the specific gravity of the lipophilic composition in the enclosing layer was set to a low specific gravity of 0.785 by using squalane as a specific gravity-adjusting agent was used for Comparative Example 5, but both of them were difficult to formulate as three-layered capsules. For formulation, a specific gravity of 0.79 or more is required.

### (Comparative Examples 6 and 7)

Capsules in which the specific gravity of the lipophilic composition in the enclosing layer was set to a high specific gravity of 1.065 by using SAIB as a specific gravity-adjusting agent were prepared for Comparative Example 6 (two-layered capsule) and Comparative Example 7 (three-layered capsule), respectively. Where the specific gravity is more than 1.05, although the formulating property of the capsule is poor, the capsule may still be produced. However, in the change over time in the enclosed water percentage, the enclosed water percentage decreased to approximately 2% after 8 weeks, which means that the enclosed water percentage was extremely poor. The data after 4 weeks and 8 weeks in the test of change over time in the enclosed water percentage show capsules of both Comparative Examples 6 and 7 each had an enclosed water percentage lower than that of the Comparative Example 2 (rubber balloon (water balloon)) and therefore, the capsules were not excellent in enclosed water stability.

### [Studies on Three-layered Dry Capsule]

It is known that a capsule containing a flavor or the like is embedded in the filter of a tobacco, and, during smoking or the like, the capsule is cracked to enjoy the fragrance or enjoy the sound and the feel generated when the capsule is cracked, and the like. As the flavors to be used, oil-soluble flavors, which are generally easy to be capsulated, have been used conventionally. Problems have been pointed out that, where a water-soluble flavor is used, even if the flavor may be capsulated, the moisture or the water-soluble component in the water-soluble flavor transfers to the capsule-shell part soon to soften the shell, whereby the sound and the feel generated when the capsule is cracked may not be enjoyed, and the like, but it was decided to conduct further studies to solve these objects.

A three-layered water-enclosed pre-capsule produced in accordance with the process in the above [Production of Water-enclosed Capsule] was dried by using a tumbler-type rotary air-blowing dryer (manufactured by Fuji Capsule Co., Ltd.) to produce a three-layered dry capsule in accordance with the prescription shown in following Table 5.

In the water-containing core, as constituent components of the water-soluble flavor, water and propylene glycol were used, and ethanol and glycerin were optionally used. As a viscosity-adding agent, hydroxypropyl cellulose was used instead of sodium alginate used in Examples described earlier.

In the enclosing layer, SAIB was used as a specific gravity-adjusting agent, and the specific gravity of the enclosing layer was set to a relatively high specific gravity of 0.94 or 0.945.

With regard to the shell layer, a prescription of a carrageenan-based shell containing carrageenan and water, a gelatin-based shell containing gelatin and water, or an agar-based shell containing agar and water, which might be used for capsules for being embedded in a tobacco filter and a cartridge for a face mask, was used.

**[Table 5]**

| | | | Example 20 | Example 21 | Example 22 | Example 23 | Example 24 | Example 25 |
|---|---|---|---|---|---|---|---|---|
| | Capsule structure | | Three-layered capsule | Three-layered capsule | Three-layered capsule | Three-layered capsule | Three-layered capsule | Three-layered capsule |
| Water-containing core | Amount filled | | 9 mg | 9 mg | 9 mg | 9 mg | 9 mg | 9 mg |
| | Propylene glycol | | 95% | 60% | 40% | 20% | 40% | 40% |
| | Ethanol | | - | 20% | 42% | 30% | 42% | 42% |
| | Glycerin | | - | - | 13% | 40% | 13% | 13% |
| | Water | | 5% | 20% | 5% | 10% | 5% | 5% |
| | Viscosity-adding agent | | Hydroxypropyl cellulose | Hydroxypropyl cellulose | Hydroxypropyl cellulose | Hydroxypropyl cellulose | Hydroxypropyl cellulose | Hydroxypropyl cellulose |
| Enclosing layer | Actual measurement value of melting point of solid oil/fat a:44.2°C | | a | a | a | a | a | a |
| | Specific gravity-adiusting agent | | SAIB | SAIB | SAIB | SAIB | SAIB | SAIB |
| | Specific gravity of enclosina layer | | 0.940 | 0.945 | 0.940 | 0.945 | 0.940 | 0.940 |
| | Shell thickness of thinnest part | | 130 µm or more | 130 µm or more | 130 µm or more | 130 µm or more | 130 µm or more | 130 µm or more |
| Shell layer | Shell | | Carrageenan-based | Carrageenan-based | Carrageenan-based | Carrageenan-based | Gelatin-based | Agar-based |
| Property of crushing of capsule with finger | Sound and feel at a time of crushing with finger | | Satisfactory | Satisfactory | Satisfactory | Satisfactory | Satisfactory | Satisfactory |
| | Enclosed water stability | 1M | Stable | Stable | Stable | Stable | Stable | Stable |

### (Confirmation of Enclosed Water Stability)

### (Examples 20 to 25)

The six types of three-layered dry capsules of Examples 20 to 25 each had a shell thickness of the thinnest part **in** the range of 130 µm to 300 µm. The specific gravity of the enclosing layer **in** which a solid oil/fat having a melting point of 44.2°C and SAIB, which was a specific gravity-adjusting agent, were used was 0.940 to 0.945, but the physical property of the enclosing layer was hard, and the capsule was a three-layered dry water-enclosed capsule. Regardless of whether the shell layer was carrageenan-based, gelatin-based, or agar-based, the capsule might be easily cracked with a finger, and the snapping sound and the feel generated when the capsule was cracked were pleasant and satisfactory. In addition, even one month (1M) after the capsule dried, the sound and the feel generated when the capsule was crushed with a finger were satisfactory, and the sound and the feel of the capsule being cracked might be enjoyed and, therefore, it was confirmed that a three-layered dry capsule having a shell thickness of the thinnest part of the enclosing layer of 130 µm or more had high enclosed water stability.

The moisture contents of the shell layers of the six types of dry capsules were each in the range of 8 to 15% as measured by using an infrared moisture meter FD-720 (manufactured by Kett Electric Laboratory Co., Ltd.) under a condition of 1 g of the shell/105°C/15 minutes. The capsules each had a thickness of the shell of 40 to 200 µm, a hardness of the shell of 0.5 to 20 N, a shell percentage of 5 to 30%, and a diameter of the capsule of 1 to 8 mm.

### [Production of Three-layered Raw Water-enclosed Capsule]

It is known that raw capsules in conventional technologies are two-layered capsules in which an oil-soluble component or a lipophilic composition is contained in a wet soft shell, and are used for cosmetics. The capsule shell is smashed by grinding the capsule when the capsule is used, whereby the contents released at the application place are mixed in a fresh state with a liquid, creamy, or half-solid cosmetic base. However, oil-soluble components and lipophilic compositions are generally used as the contents, and no raw capsule containing moisture or a water-soluble component is known. Therefore, it was decided to conduct studies on a three-layered raw capsule having a water-containing core containing water assuming the usage as described above (Examples 26 to 30).

A water-enclosed pre-capsule for each prescription shown in following Table 6 was prepared in accordance with the process in the above [Production of Water-enclosed Capsule] and washed with running water, and then the three-layered raw capsule was immersed in a storing liquid (storing aqueous solution/colorless) containing a preservative (phenoxyethanol) and an antibacterial agent (1,3-butylene glycol).

In the water-containing core, Brilliant Blue FCF was added to the water as a dye for visual confirmation of the transfer of the dye in the water-containing core in the water-resistance test, and sodium alginate was used as a viscosity-adding agent.

In the enclosing layer, MCT (COCONARD MT, manufactured by Kao Corporation) was used as a liquid oil/fat, together with a solid oil/fat, resulting in a relatively high specific gravity of the enclosing layer. In Examples 27 to 30, SAIB was used as a specific gravity-adjusting agent, and the specific gravity of the enclosing layer was set to a high specific gravity of 1.01 and 1.00. The shell thicknesses of the thinnest parts were 150 µm or more.

With regard to the shell layer, a prescription of a carrageenan-based shell containing carrageenan and water, a gelatin-based shell containing gelatin and water, or an agar-based shell containing agar and water was used.

As a method for evaluating the water resistance, a method in which the transfer of the dye in the water-containing core when the three-layered raw capsule was immersed for storage in a storing liquid (storing aqueous solution/colorless) containing a preservative (phenoxyethanol) and an antibacterial agent (1,3-butylene glycol) was visually confirmed was used. If the moisture or the water-soluble component of the water-containing core is sealed and stably retained in each of the three-layered raw capsule, the storing liquid is in the colorless state and there is no change in the color tone, and it may be confirmed that the capsule has water resistance. On the one hand, when the moisture or the water-soluble component in the water-containing core has transferred to the storing liquid part through the shell, the storing liquid is colored blue, and it may be visually confirmed that the moisture or the water-soluble component of the water-containing core is not stably retained and the capsule lacks in water resistance. The results are shown in following Table 6.

**[Table 6]**

| | | | Example 26 | Example 27 | Example 28 | Example 29 | Example 30 |
|---|---|---|---|---|---|---|---|
| | Capsule structure | | Three-layered capsule | Three-layered capsule | Three-layered capsule | Three-layered capsule | Three-layered capsule |
| Water-containing core | Amount filled | | 8 mg | 8 mg | 8 mg | 8 mg | 8 mg |
| | Water content | | 98.5% | 98.5% | 98.5% | 98.5% | 98.5% |
| | Viscosity-adding agent | | Sodium alginate | Sodium alginate | Sodium alginate | Sodium alginate | Sodium alginate |
| | Coloring to water | | Brilliant Blue FCF | Brilliant Blue FCF | Brilliant Blue FCF | Brilliant Blue FCF | Brilliant Blue FCF |
| Enclosing layer | Actual measurement value of melting point of solid oil/fat | | a | a | - | - | - |
| | a:44.2°C | | - | - | b | b | b |
| | b:35.3°C | | | | | | |
| | Liquid oil/fat | | MCT | MCT | MCT | MCT | MCT |
| | Specific gravity-adjusting agent | | - | SAIB | SAIB | SAIB | SAIB |
| | Specific gravity of enclosing layer | | 0.930 | 1.010 | 1.000 | 1.000 | 1.000 |
| | Shell thickness of thinnest part | | 150 µm or more | 150 µm or more | 150 µm or more | 150 µm or more | 150 µm or more |
| | State of enclosing layer | | Raw | Raw | Raw | Raw | Raw |
| Shell layer | Shell | | Agar-based | Agar-based | Agar-based | Gelatin-based | Carrageenan-based |
| | State of shell layer | | Raw | Raw | Raw | Raw | Raw |
| Water resistance | Transfer of dye to storing liquid part | | None | None | None | None | None |
| | Enclosed water stability | 3M | Stable | Stable | Stable | Stable | Stable |

### (Examples 26 to 30)

### (Confirmation of Water Resistance)

The three-layered capsules of Examples 26 to 30 were each a three-layered raw water-enclosed capsule having a shell thickness of the thinnest part of the enclosing layer of 150 µm or more and a specific gravity of the enclosing layer in which MCT was used as a liquid oil/fat of 0.930 to 1.010. It was confirmed that there was no transfer of the dye to the storing liquid part even three months (3M) after the dried three-layered raw capsule was immersed in a storing liquid and, therefore, the capsule had high water resistance. In addition, these capsules were able to be easily ground with a finger for use. The measured values of the moisture content of the shell of the three-layered raw capsule include 20.4% for a carrageenan-based shell capsule, 49.6% for a gelatin-based shell, and 97.5% for an agar-based shell capsule, and it was determined that the shells having these moisture contents were each soft and therefore the capsules were raw capsules.

### [Studies on Water Resistance of Capsule]

As shown in following Table 7 (Examples 31 to 35), two-layered solid capsules or three-layered capsules having various shell thicknesses of the thinnest parts of the enclosing layers were produced.

The water resistance of the water-enclosed capsule produced in accordance with the prescription shown in following Table 7 was evaluated. That is, water was put in a 20 mL glass vial, and one capsule was immersed in the water and then stored in the state where the glass vial was covered with a lid at room temperature. Whether or not the dye in the water-containing core of the capsule being immersed in the water was leaked out of the capsule over time was visually confirmed after each of 4 weeks, 8 weeks, 12 weeks, and 16 weeks.

**[Table 7]**

| | | | Comparative Example 8 | Example 31 | Example 32 | Example 33 | Example 34 | Example 35 |
|---|---|---|---|---|---|---|---|---|
| | Capsule structure | | Two-layered capsule | Two-layered capsule | Two-layered capsule | Three-layered capsule | Two-layered capsule | Two-layered capsule |
| Water-containing core | Amount filled | | 100 mg | 100 mg | 100 mg | 100 mg | 100 mg | 100 mg |
| | Water content | | 98.5% | 98.5% | 98.5% | 98.5% | 98.5% | 98.5% |
| | Viscosity-adding agent | | Sodium alginate | Sodium alginate | Sodium alginate | Sodium alginate | Sodium alginate | Sodium alginate |
| Enclosing layer | Actual measurement value of melting point of solid oil/fat a:44.2°C | | a | a | a | a | a | a |
| | Specific gravity-adjusting agent | | - | - | - | - | SAIB | SAIB |
| | Specific gravity of enclosing layer | | 0.885 | 0.885 | 0.885 | 0.885 | 0.940 | 1.030 |
| | Shell thickness of thinnest part | | 60 µm | 200 µm | 500 µm or more | 500 µm or more | 500 µm or more | 500 µm or more |
| Shell layer | Shell | | None | None | None | Gelatin-based | None | None |
| Change over time in leakage of dye in water-containing core | Allowed to stand under closed atmosphere at 22°C | 4W | 4/10 | 0/10 | 0/5 | 0/5 | 0/5 | 0/5 |
| | | 8W | 10/10 | 0/10 | 0/5 | 0/5 | 0/5 | 0/5 |
| | Number of occurrence of leakage/number of samples | 12 W | - | 0/10 | 0/5 | 0/5 | 0/5 | 0/5 |
| | | 16 W | - | 0/10 | 0/5 | 0/5 | 0/5 | 0/5 |
| | Water resistance of capsule | | No | Yes | Yes | Yes | Yes | Yes |

### (Results)

As is clear from Table 7, Comparative Example 8 had a shell thickness of the thinnest part of the enclosing layer of 60 µm, but the dye leaked out of all capsules after 8 weeks, resulting in failure in obtaining water resistance. On the one hand, Example 31 was a two-layered solid water-enclosed capsule having a shell thickness of the thinnest part of 200 µm or more and Example 32 was a two-layered solid water-enclosed capsule having a shell thickness of the thinnest part of 500 µm or more, and the water in the glass vials had not been colored blue even after 16 weeks and, therefore, the capsules were each a capsule having no leakage of the dye and having water resistance. In the three-layered dry capsule of Example 33 including an enclosing layer having a shell thickness of the thinnest part of 500 µm or more, the gelatin shell layer swelled over time, but the capsule had no leakage of the dye in the water-containing core and therefore had water resistance. Examples 34 and 35 are two-layered solid water-enclosed capsules in which the specific gravities of the enclosing layers were set to 0.940 and 1.030, respectively, and satisfactory water-resistant capsules were obtained.

### Industrial Applicability

The water-enclosed capsule of the present invention may be utilized as a water-enclosed capsule that may stably retain hydrophilic volatile substances over a long period of time in the fields of medicines; quasi-drugs; cosmetics; foods; beverages; dairy products; seasonings; supplements; pet foods; daily necessities for households; luxury items such as tobaccos and substitutes for tobaccos; fertilizers; feed; industrial products such as cleaning agents; and other fields.

## Claims

1. A water-enclosed capsule comprising:
a water-containing core containing water; and
an enclosing layer disposed on an outside of the water-containing core,
wherein the enclosing layer consists of a lipophilic composition having a specific gravity of 0.79 to 1.05, and
wherein a thinnest part of a shell thickness of the enclosing layer is 80 µm or more.

2. The water-enclosed capsule according to claim 1, wherein the water-containing core contains a viscosity-adding agent for imparting viscosity to the water.

3. The water-enclosed capsule according to claim 1, wherein the lipophilic composition contains a specific gravity-adjusting agent for adjusting the specific gravity of the lipophilic composition to 0.79 to 1.05.

4. The water-enclosed capsule according to claim 1, wherein a melting point of the lipophilic composition is 16°C or more.

5. The water-enclosed capsule according to claim 1, wherein the lipophilic composition contains a solid oil/fat having a melting point of 30 to 70°C.

6. The water-enclosed capsule according to claim 1, wherein the lipophilic composition contains a liquid oil/fat having a melting point of less than 20°C.

7. The water-enclosed capsule according to claim 1, wherein a shell layer is further provided on an outside of the enclosing layer.

8. The water-enclosed capsule according to claim 7, wherein a moisture content of the shell layer is 20% or more.

9. The water-enclosed capsule according to claim 7, wherein a moisture content of the shell layer is less than 20%.

10. A method for producing the water-enclosed capsule according to any one of claims 1 to 9, the method comprising:
forming a three-layered liquid drop by discharging a water-containing core from an inner nozzle, an enclosing layer from an intermediate nozzle, and a shell layer from an outer nozzle by using a triple concentric nozzle, and
forming a water-enclosed pre-capsule by contacting the three-layered liquid drop with a cooling solvent.

11. The method for producing the water-enclosed capsule according to claim 10, wherein, before drying the water-enclosed pre-capsule, the shell layer is stripped off and eliminated to leave two layers of the water-containing core containing water and the enclosing layer disposed on an outside of the water-containing core.

12. The method for producing the water-enclosed capsule according to claim 10, wherein a three-layered water-enclosed raw capsule is prepared without drying the water-enclosed pre-capsule.

13. The method for producing the water-enclosed capsule according to claim 10, wherein a three-layered water-enclosed dry capsule is prepared by further undergoing a step of drying the water-enclosed pre-capsule.
